Europäisches Patentamt

European Patent Office

Office européen des brevets

(19)

(11) Veröffentlichungsnummer: **0 212 217**
**B1**

(12) # EUROPÄISCHE PATENTSCHRIFT

(45) Veröffentlichungstag der Patentschrift:
04.01.89

(51) Int. Cl.⁴: **C07C 27/26**

(21) Anmeldenummer: 86109482.9

(22) Anmeldetag: **11.07.86**

(54) **Verfahren zur Stabilisierung von sauren Waschwässern.**

(30) Priorität: **24.07.85 DE 3526412**

(43) Veröffentlichungstag der Anmeldung:
**04.03.87 Patentblatt 87/10**

(45) Bekanntmachung des Hinweises auf die Patenterteilung:
**04.01.89 Patentblatt 89/1**

(84) Benannte Vertragsstaaten:
**BE CH DE FR IT LI NL**

(56) Entgegenhaltungen:
**EP-A- 0 133 696**
**CH-A- 630 332**
**DE-B- 1 046 610**
**DE-B- 1 518 255**
**DE-B- 1 643 692**
**DE-B- 2 061 113**
**US-A- 4 341 907**
**US-A- 4 465 861**

(73) Patentinhaber: **BASF Aktiengesellschaft,
Carl-Bosch-Strasse 38, D-6700 Ludwigshafen(DE)**

(72) Erfinder: **Herrmann, Günther, Dr., Haeusserstrasse 51,
D-6900 Heidelberg(DE)**
Erfinder: **Lucas, Ekhart, Dr., Burgunderweg 7,
D-6706 Wachenheim(DE)**

# Beschreibung

Gegenstand der Erfindung ist ein Verfahren zur Stabilisierung von sauren Waschwässern aus der Cyclohexanoxidation.

Bei der Oxidation von Cyclohexan mit molekularem Sauerstoff oder solchen enthaltenden Gasen entstehen neben Cyclohexanol und Cyclohexanon saure und andere Nebenprodukte, die aus dem Reaktionsgemisch vor der Gewinnung der Wertprodukte abgetrennt werden müssen. Solche wasserlösliche Nebenprodukte werden durch Auswaschen des Oxidationsgemisches mit Wasser abgetrennt. Die sauren Waschwässer enthalten Carbonsäuren, wie Adipinsäure, Hydroxicarbonsäuren und andere, nicht identifizierte Bestandteile. Diese sauren Waschwässer werden üblicherweise weiter verarbeitet, um z.B. die darin enthaltene Adipinsäure zu gewinnen. Hierzu werden die sauren Waschwässer in großen Behältern gesammelt und an den Ort der Weiterverarbeitung transportiert. Um ein Abscheiden von Feststoffen zu verhindern, hält man solche Lösungen auf erhöhter Temperatur, z.B. auf 80°C. Es hat sich herausgestellt, daß solche saure Waschwässer je kg der Lösung 0,4 bis 2,5 l eines überwiegend aus Wasserstoff bestehenden Gases entbinden. Durch Mischen solcher Gase mit Luftsauerstoff entstehen explosive Gemische, die sowohl die Lagerung als auch den Transport der sauren Waschwässer erheblich gefährden.

Aus der CH-A 630 332 ist zwar bekannt, daß man Cyclohexanoxidationsgemische unter Zusatz von Schwermetallverbindungen, z.B. Cobaltverbindungen, mit Alkalilauge behandelt. Vorteilhaft werden die zu behandelnden Cyclohexanoxidationsgemische mit Wasser gewaschen, um den größten Teil an sauren Nebenprodukten zu entfernen. Es ist jedoch dort kein Hinweis zu entnehmen, wie solche sauren Waschwässer stabilisiert werden können, um die Bildung von Wasserstoff zu unterbinden.

Es war deshalb die technische Aufgabe gestellt, ein Verfahren zur Stabilisierung von sauren Waschwässern aus der Cyclohexanoxidation zur Verfügung zu stellen, bei dem die Bildung von Wasserstoff enthaltenden Gasen verhindert wird.

Diese Aufgabe wird gelöst in einem Verfahren zur Stabilisierung von sauren Waschwässern, die durch Auswaschen von Oxidationsgemischen aus der Cyclohexanoxidation mit Wasser erhalten worden sind, wobei man die sauren Waschwässer in Gegenwart von wasserlöslichen Vanadiumverbindungen auf eine Temperatur von 50 bis 130°C erhitzt.

Das neue Verfahren hat den Vorteil, daß die Bildung von Wasserstoff unterbunden wird und die Gefahrenquelle bei der Lagerung und bei dem Transport solcher sauren Waschwässer beseitigt wird.

Erfindungsgemäß geht man von sauren Waschwässern aus, die durch Auswaschen von Oxidationsgemischen aus der Cyclohexanoxidation mit Wasser erhalten worden sind. Solche Oxidationsgemische gewinnt man durch Oxidation von Cyclohexan mit molekularem Sauerstoff oder solchen enthaltenden Gasen, z.B. Luft, in flüssiger Phase bei Temperaturen von 130 bis 200°C und unter Drücken von 5 bis 25 bar, gegebenenfalls unter Mitverwendung von Katalysatoren. Die Oxidation wird in der Regel in mehreren Stufen, z.B. in 2 bis 5 Stufen durchgeführt. Das so erhaltene Oxidationsgemisch wird entweder nach jeder Stufe, zwischendurch oder nach der letzten Stufe insgesamt mit Wasser ausgewaschen, um die sauren Bestandteile zu entfernen. Die Wasserwäsche erfolgt in der Regel bei einer Temperatur von 80 bis 140°C und unter einem Druck von 10 bis 25 bar. Das saure Waschwasser wird dann vom verbleibenden Cyclohexanoxidationsgemisch mit üblichen Methoden, z.B. Dekantieren, abgetrennt. Typische saure Waschwässer enthalten insgesamt z.B. 10 bis 20 Gew.% Dicarbonsäuren, wie Adipinsäure sowie Monocarbonsäuren, wie Valeriansäure und Oxicapronsäure und andere nicht identifizierte Nebenprodukte. Im allgemeinen haben die sauren Waschwässer eine Säurezahl von 50 bis 150.

Erfindungsgemäß werden den sauren Waschwässern wasserlösliche Vanadiumverbindungen zugesetzt. Geeignete Vanadiumverbindungen sind beispielsweise Natriumvanadat, Kaliumvanadat, Ammoniumvanadat, ferner Kaliummetavanadat. Besonders bewährt hat es sich, eine Lösung von Vanadinpentoxid in Natronlauge, z.B. eine 2- bis 6gew.%ige Lösung von Vanadinpentoxid in 10- bis 20gew.%iger Natronlauge, zu verwenden. Vorteilhaft setzt man den sauren Waschwässern 0,1 bis 100 ppm, insbesondere 0,5 bis 50 ppm, wasserlösliche Vanadiumverbindungen berechnet als Vanadium zu. Es hat sich besonders bewährt, wenn man die wasserlöslichen Vanadiumverbindungen den sauren Waschwässern unmittelbar nach deren Abtrennung vom Cyclohexanoxidationsgemisch zusetzt.

Die Behandlung führt man bei einer Temperatur von 50 bis 130°C, insbesondere bei einer Temperatur von 70 bis 120°C, durch. Hierbei hält man vorteilhaft eine Verweilzeit von 15 bis 90 Minuten bei den angegebenen Temperaturen ein.

Wäßrige Lösungen, die nach dem Verfahren der Erfindung erhalten werden, eignen sich z.B. für die Herstellung von Hexandiol.

Das Verfahren nach der Erfindung sei an folgenden Beispielen veranschaulicht.

Beispiel 1

Saures Waschwasser, das durch Waschen eines Cyclohexanoxidationsgemisches mit Wasser bei einer Temperatur von 120°C unter einem Druck von 15 bar erhalten wurde und eine Säurezahl von 90 aufweist, wird mit Natriumvanadat versetzt, so daß der Vanadiumgehalt 10 ppm beträgt. Die Lösung wird für einen Zeitraum von 30 Minuten auf 100°C erhitzt. Aus der so behandelten Lösung entweicht auch nach längerem Lagern bei 90°C kein Wasserstoff.

Beispiel 2

Zu einem sauren Waschwasser, wie in Beispiel 1 beschrieben, setzt man eine solche Menge Kaliummetavanadat zu, daß der Gehalt an Vanadium 20 ppm beträgt. Die Lösung wird bei einer Temperatur von 100° C durch eine zweistufige Rührkesselkaskade geleitet, wobei die Verweilzeit in jeder Stufe 30 Minuten beträgt. An der so erhaltenen Lösung ist keine Wasserstoffabgabe zu beobachten.

Vergleichsbeispiel 1

Man verwendet das in Beispiel 1 beschriebene saure Waschwasser und hält dieses auf einer Temperatur von 80°C. Im Verlaufe von 20 bis 40 Stunden werden 0,4 bis 2,5 l Gas je kg Lösung entbunden, das überwiegend aus Wasserstoff besteht und geringe Mengen an Kohlenmonoxid und Kohlendioxid enthält.

Vergleichsbeispiel 2

Man verwendet das in Beispiel 1 beschriebene saure Waschwasser und versetzt es mit Kobaltsulfat, Mangansulfat oder Kaliumchromat, so daß der Schwermetallgehalt jeweils 20 ppm beträgt. Die jeweilige Lösung wird für einen Zeitraum von 30 Minuten auf 100°C erhitzt.

Aus der so behandelten Lösung entweicht beim Lagern bei 90°C Wasserstoff.

Patentansprüche

1. Verfahren zur Stabilisierung von sauren Waschwässern, die durch Auswaschen von Oxidationsgemischen aus der Cyclohexanoxidation mit Wasser erhalten worden sind, dadurch gekennzeichnet, daß man die sauren Waschwässer in Gegenwart von wasserlöslichen Vanadiumverbindungen auf eine Temperatur von 50 bis 130°C erhitzt.

2. Verfahren nach Anspruch 1, dadurch gekennzeichnet, daß man 0,1 bis 100 ppm wasserlösliche Vanadiumverbindungen berechnet als Vanadium zugibt.

3. Verfahren nach den Ansprüchen 1 und 2, dadurch gekennzeichnet, daß man eine Lösung von Vanadiumpentoxid in Natronlauge verwendet.

4. Verfahren nach den Ansprüchen 1 bis 3, dadurch gekennzeichnet, daß man die wasserlöslichen Vanadiumverbindungen unmittelbar nach Abtrennung des sauren Waschwassers von dem Cyclohexanoxidationsgemisch zusetzt.

Claims

1. A process for stabilizing an aqueous acidic scrubbing liquor obtained when an oxidation mixture from the oxidation of cyclohexane is scrubbed with water, wherein the aqueous acidic scrubbing liquor is heated at from 50 to 130°C in the presence of a water-soluble vanadium compound.

2. A process as claimed in claim 1, wherein from 0.1 to 100 ppm, calculated as vanadium, of a water-soluble vanadium compound is added.

3. A process as claimed in either of claims 1 and 2, wherein a solution of vanadium pentoxide in sodium hydroxide solution is used.

4. A process as claimed in any of claims 1 to 3, wherein the water-soluble vanadium compound is added directly after the aqueous acidic scrubbing liquor has been separated off from the cyclohexane oxidation mixture.

Revendications

1. Procédé de stabilisation d'eaux de lavage qui ont été obtenues par lavage avec de l'eau de mélanges d'oxydation provenant de l'oxydation du cyclohexane, caractérisé par le fait que l'on chauffe les eaux de lavage acides à une température de 50 à 130°C en présence de composés de vanadium solubles dans l'eau.

2. Procédé selon la revendication 1, caractérisé par le fait que l'on ajoute 0,1 à 100 ppm de composés de vanadium solubles dans l'eau, calculés en tant que vanadium.

3. Procédé selon les revendications 1 et 2, caractérisé par le fait que l'on utilise une solution de pentoxyde de vanadium dans une lessive de soude.

4. Procédé selon les revendications 1 à 3, caractérisé par le fait que l'on ajoute les composés de vanadium solubles dans l'eau directement après séparation de l'eau de lavage acide du mélange d'oxydation du cyclohexane.